# EUROPÄISCHE PATENTANMELDUNG

(11) **EP 1 531 178 A1**
(43) Veröffentlichungstag der Anmeldung: **18.05.2005**
(21) Anmeldenummer: 03026130.9
(22) Anmeldetag: 13.11.2003
(51) Int. Cl.: C12N 1/16, C02F 11/04, C02F 3/34

(54) **Verfahren zur Gewinnung und Weiterverarbeitung von Hefebiomasse**

(71) Anmelder: Fritz Köster Handelsgesellschaft AG, 20148 Hamburg (DE)
(72) Erfinder: Jackisch, Thomas, 29664 Walsrode-Düshorn (DE)
(74) Vertreter: Glawe, Delfs, Moll & Partner

(57) **Zusammenfassung**

Gegenstand der Erfindung ist ein Verfahren zur Verarbeitung von Prozeßwasser, das als Hefefermentationssubstrat geeignet ist. Das Prozeßwasser wird aerob mit substratspezifischen Hefepopulationen fermentiert und das Gemisch anschließend in Hefebiomasse und Fermentationslösung separiert. Die mit organischen Rückständen belastete Fermentationslösung wird zur Erzeugung von Biogas weiterverwendet. Die aus dem Biogas gewonnene Energie wird in den Hefebiomassengewinnungs- und weiterverarbeitungsprozeß zurückgeführt, insbesondere zur Trocknung der Hefebiomasse verwendet.

## Beschreibung

Die Erfindung betrifft ein Verfahren zur Gewinnung und Weiterverarbeitung von Hefebiomasse aus Prozeßwasser, das als Hefefermentationssubstrat geeignet ist.

Biotechnologische Verfahren zur Gewinnung von Hefebiomasse mittels aerober Fermentation sind aus offenkundiger Vorbenutzung bekannt.

Hefebiomasse wird unter anderem verwendet als Futtermittel, Grundstoff für Aromaextrakte, Geschmacksverstärker und Nahrungsergänzungsmittel, als auch Mineral- bzw. Vitaminträger und Rohstofflieferant für Pharmaka.

Ein Nährmedium, das im Stand der Technik zur Gewinnung von Hefebiomasse Verwendung findet, ist Molke.

Eine zusammengefaßte Darstellung zur biotechnologischen Nutzung von Molke zur Gewinnung von Biomasse wird in den Übersichtsartikel Gonzalez Siso, M. I. The biotechnological utilization of cheese whey: A review Bioresource Technology 57, (1996), 1-11 gegeben. Hefestämme, die in solchen Prozessen eingesetzt werden sind z. B. Candida sp., Klyveromyces sp., Torulopsis sp., Debaromyces sp. und Saccharomyces sp. nach enzymatischer Spaltung der Lactose. Das mit Hefepopulationen fermentierte Prozeßwasser wird im Stand der Technik nach zwei bekannten Verfahren aufgearbeitet. Entweder werden die Hefen und die übrigen Prozeßwasserbestandteile zusammen konzentriert und getrocknet, oder die Hefen werden von dem übrigen Prozeßwasserbestandteilen abgetrennt und anschließend getrocknet.

Nach der letzten Methode entsteht nach Abtrennung der Hefe von den Prozeßwasserbestandteilen eine Fermentationslösung, die beispielsweise wie in US-A-4,192,918 offenbart, in das öffentliche Abwassernetz abgelassen wird. Das ist problematisch, da sich der Gehalt an organischen Material in der Fermentationslösung (Abwasser nach US-A-4,192,918) häufig immer noch als inakzeptabel hoch erweist. Beim Ablassen der Fermentationslösung (Abwasser) in die Umwelt bauen bestimmte aerobe Bakterien die komplexen organischen Verbindungen umweltneutral zu beispielsweise CO₂, NO⁻₃-Ionen und SO₄⁻²-Ionen ab. Dabei wird dem Wasser jedoch Sauerstoff entzogen. Bei besonders hoher Belastung des Abwassers mit organischen Materialien können sich für die Umwelt äußerst giftige Gase wie CH_{4.}, NH₃ und H₂S bilden, die neben den fehlenden Sauerstoff ein Leben für bestimmte Tiere und Pflanzen ausschließen und das ökologische Gleichgewicht erheblich stören. Es ist außerdem bekannt, daß für das Wachstum der Hefe zusätzlich Supplemente dem Prozeßwasser zugegeben werden, wie z. B. Amoniumsalze, deren Anionen nach Assimilation des freien Ammoniaks durch die Hefen in Form freier Säuren vorliegen. Diese anorganischen Säuren stellen aber eine zusätzliche Belastung des Abwassers da.

Neben dem anfallenden Abwasser wird die Gewinnung von Hefebiomasse durch den notwendigen hohen energetischen Aufwand, besonders bei der Produktion von Trockenhefe, wirtschaftlich erheblich belastet.

Neben der Gewinnung von Hefebiomasse durch aerobe Fermentation ist aus offenkundiger Vorbenutzung die Energiegewinnung durch anaerobe Fermentation von Biomasse, beispielsweise Gülle, Festmist, Abfällen aus der Lebensmittelindustrie oder nachwachsenden Rohstoffen, in Biogasanlagen bekannt, wie sie hauptsächlich in Kläranlagen und Deponien eingesetzt wird. Dabei entstehen methanhaltige Gase, die sich universell, z. B. zum Heizen, zum Trocknen oder zur Stromerzeugung einsetzen lassen.

Der Erfindung liegt die Aufgabe zugrunde, ein Verfahren zur Verarbeitung von Prozeßwasser unter Gewinnung von Hefebiomasse zu schaffen, bei dem praktisch kein Abwasser, welches die Umwelt belastet, anfällt und das erlaubt, die bei der Biomasse-Gewinnung und Weiterverarbeitung anfallenden hohen Energiekosten zu verringern.

Die Erfindung löst diese Aufgabe durch ein Verfahren zur Verarbeitung von Prozeßwasser, das als Hefefermentationssubstrat geeignet ist, mit den Schritten:
a) Aerobe Fermentation des Prozeßwassers mit substratspezifischen Hefepopulationen,
b) Separierung des Fermentationsgemisches aus Schritt a) in Hefebiomasse und Fermentationslösung,
c) Verwendungen der in Schritt b) erhaltenen Fermentationslösung bei der Erzeugung von Biogas,
d) Verwendung des in Schritt c) erzeugten Biogases zur Gewinnung von thermischer und/oder elektrischer Energie,
e) zumindest teilweise Nutzung der in Schritt d) gewonnen Energie bei der Gewinnung und/oder Weiterverarbeitung der in Schritt b) separierten Hefebiomasse.

Besonders bevorzugte Ausführungsformen der Erfindung sind in den Unteransprüchen offenbart.

Zunächst sein einige im Rahmen der Erfindung verwendete Begriffe erläutert.

Der Begriff Biomasse bezeichnet die Gesamtheit von lebendem, totem und zersetztem organischem Material. Vorliegende Erfindung verwendet den Begriff Hefebiomasse, wenn die Biomasse aus Hefezellen gebildet wird. Bei der Hefebiomassen-Gewinnung und Weiterverarbeitung ist somit der Mikroorganismus Hefe selbst die gewonnene Biomasse und die aus der Hefe erhaltenen Stoffe, beispielsweise Hefelysat oder Hefeextrakt, die weiterverarbeitete Hefebiomasse.

Der Begriff Prozeßwasser bezeichnet jegliche Flüssigkeit, die als Hefefermentationssubstrat, d. h. als Nährmedium für Hefepopulationen, geeignet ist.

Nach einer bevorzugten Ausführungsform der Erfindung enthält das Prozeßwasser Lactose. Beispielsweise kann Molke oder aus Molke erhaltenes Molkepermeat als Prozeßwasser dienen. Molkepermeat ist ein Nebenprodukt der Ultrafiltration von Molke bei der Herstellung eines Molke-Protein-Konzentrats. Die Molke oder das Molkepermeat kann aufkonzentriert sein, d. h. ihr wurde Flüssigkeit entzogen, wodurch der Lactosegehalt, der bei der Molke üblicherweise in einem Bereich von 4,5 - 5 Gew.%, der vom Molkepermeat bei 7,5 - 9,0 Gew.% liegt, höher ausfallen kann. Gemäß einer bevorzugten Ausführungsform der Erfindung liegt der Lactosegehalt des Prozeßwassers zwischen 0,4 Gew.% - 40 Gew.%, vorzugsweise 2 Gew.% - 12 Gew.%, besonders bevorzugt 3,5 Gew.% - 9 Gew.%.

Die Auswahl der zu Fermentation einzusetzenden Hefepopulationen richtet sich danach, welche Populationen bestmöglich zu Verwertung der im Prozeßwasser vorhandenen primären Nährstoffe geeignet sind. Als Hefepopulationen sind erfindungsgemäß besonders die Gattungen Candida, Kluyveromyces, Debaromyces, Trichosporon, Pichia, Saccharomyces und Torolopsis geeignet. Für lactosehaltiges Prozeßwasser wie Molke oder Molkepermeat ist z. B. die Gattung Kluyveromyces, beispielsweise die Spezien Kluyveromyces fragilis und Kluyveromyces marxianus vorteilhafterweise geeignet. Stärkehaltiges Prozeßwasser ist beispielsweise bevorzugtes Substrat für die Gattung Candida, sacharosehaltiges Prozeßwasser für die Gattung Saccharomyces. Enthält das Prozeßwasser mehrere Substrate, die von verschiedenen Hefegattungen bzw. Spezien unterschiedlich effizient verwertet werden, ist es von Vorteil, Hefe-Mischpopulationen, abgestimmt auf die Substrate des Prozeßwassers, bei der Fermentation einzusetzen.

Eine Ausführungsform der Erfindung sieht vor, daß das Prozeßwasser sterilisiert wird, bevor es mit den Hefepopulationen fermentiert wird. Die Sterilisation dient dazu, Mikroorganismen, die das Hefewachstum behindern könnten, abzutöten bzw. deren Zahl zu vermindern. Dafür geeignete und aus der Lebensmitteltechnologie bekannte Verfahren sind beispielsweise die thermischen Verfahren: Pasteurisation, Tyndallisation und Uperisation sowie die Hochdruckbehandlung. Die Pasteurisation wird in Nieder- und Hochpasteurisierung unterschieden. Eine typische Niederpasteurisierung erfolgt in dreißig Minuten bei 60,5 °C (Dauerpasteurisierung) und 15 sec. bei 71,0°C. Bei der Hochpasteurisierung erfolgt typischerweise ein kontinuierlicher Betrieb bei 80-85°C für wenige Sekunden. Bei diesem Verfahren ist eine Keimzahl unter 20-30.000/ml erreichbar. Bei der Tyndallisation erfolgt ein mehrmaliges Erhitzen auf 70-80°C mit dazwischenliegenden Abkühlungsintervallen von bis zu zwanzig Stunden in denen die Sporen auskeimen können bevor sie beim folgenden Erhitzen abgetötet werden. Die Uperisation erfolgt bei 100-150°C wobei dem vorgewärmten Gut eine Dampfinjektion zugeführt und beim Rückkühlen im Vakuum wieder entfernt wird. Der Vorteil der Uperisation, wie es beispielsweise zur Sterilisation von Milch zu H-Milch angewandt wird, liegt darin, daß die H-Milch nicht nur keimarm, sondern keimfrei ist. Keimfrei heißt in diesem Falle, daß sie eine 15-tägige Lagerung bei 30°C überstehen muß. Die Hochdruckbehandlung kann bei sehr geringen Starttemperaturen erfolgen, als auch zur weiteren Erhöhung der Temperatur während der Pasteurisierung eingesetzt werden. Der Einfluß des Druckes bewirkt eine Temperaturerhöhung, die gleichzeitig im gesamten Sterilisationsgut wirkt. Der applizierte Druck sorgt für die Reduzierung der Mikroorganismen.

Für eine optimale Nutzung des Substrates im Prozeßwasser durch die Hefen kann das Prozeßwasser mit Stickstoff- und Phosphorquellen, sowie mit Spurenelementen/Vitaminen für die Hefe supplementiert werden. Hierfür werden verschiedene Substanzen wie z. B. : Diammoniumhydrogenphosphat, Harnstoff, Hefeextrakt, Ammoniumsulfat und Kaliumdihydrogenphosphat eingesetzt.

Das erfindungsgemäße aerobe Fermentationsverfahren wird gemäß einer Ausführungsform der Erfindung in einer kontinuierlichen Kultur durchgeführt. Eine kontinuierliche Fermentation im Sinne vorliegender Erfindung ist eine Fermentation bei dem kontinuierlich ein Abzug von Fermentationsgemisch und dessen Ersatz durch äquivalente Mengen frischen Prozeßwassers erfolgt. Eine neue Beimpfung des frischen Prozeßwassers mit Hefepopulationen ist nicht erforderlich, kann aber, beispielsweise bei einem starken Abfall der Populationsdichte der Hefe, sinnvoll sein, um die Kultur möglichst schnell in die bevorzugte Phase des Wachstums zurückzuführen. Die kontinuierliche Fermentation erfolgt bei vorliegender Erfindung vorzugsweise bei einer Temperatur von 20-40°C weiter, vorzugsweise 25-35°C, besonders bevorzugt 29-31°C und/oder einem pH < 4 und/oder einer Durchflußrate von 1-48 Stunden, bevorzugt 5-24 Stunden, besonders bevorzugt 8-12 Stunden. Der pH-Wert < 4 erweist sich als besonders bevorzugt, da in diesem Bereich das Wachstum der mit den Hefen konkurrierenden Bakterien stark gehemmt ist.

Des weiteren ist bei den erfindungsgemäßen Verfahren die diskontinuierliche (Batch-)Fermentation vorgesehen. Bei der diskontinuierlichen Fermentation findet kein kontinuierlicher Entzug vom Fermentationsgemisch aus der Kultur unter Zugabe von frischer Nährlösung für die Hefepopulation statt. Eine kontinuierliche Batch-Kultur durchläuft nach der Animpfung der Nährlösung mit den Hefepopulationen die lag-Phase, bei denen sich die Hefezellen an die jeweilige Umgebung anpassen und der Enzymapparat der Hefe zur Verwertung der jeweilige Substrate induziert wird, nachfolgend eine Beschleunigungsphase, die mit der ersten Teilung der Hefezellen beginnt und endet, sobald die Wachstumsgeschwindigkeit den maximalen Wert erreicht hat. Dem folgt die Exponentialphase, in der die Kultur die maximale Wachstumsgeschwindigkeit zeigt bevor sich durch Nährstofferschöpfung, Anhäufung von Stoffwechselprodukten und die Populationsdichte bedingt die Verzögerungsphase am Ende der Expondentialphase eintritt. Wird in einer Batch-Kultur nach Beginn der Verzögerungsphase ein bestimmter Anteil der Kultur abgezogen und durch eine äquivalente Menge frischen Prozeßwassers ersetzt, wobei dieser Vorgang beliebig oft wiederholt werden kann, spricht man von einer semikontinuierlichen Kultur.

Um die günstigen Bedingungen für das Hefewachstum bei der aeroben Fermentation aufrechtzuerhalten, wird in der Biotechnologie meistens mit Rührfermentatoren gearbeitet. Diese Fermentatoren gewährleisten die Durchmischung des Mehrphasensystems (Hefe, evtl. unlösliches Substrat = feste Phase; Nährlösung, evtl. zudosierte Lauge bzw. Säure = flüssige Phase; Luft = Gas-Phase) als Voraussetzung für gutes Wachstum der Hefen.

Der aeroben Fermentation des Prozeßwassers mit den Hefepopulationen schließt sich erfindungsgemäß die Separierung des Gemisches in Hefebiomasse und von Hefezellen abgereicherte Fermentationslösung an. Bei der Separierung des Fermentationsgemisches durch Zentrifugation ist der Überstand die Fermentationslösung. Weitere, aus dem Stand der Technik bekannte Separierungsmethoden, beispielsweise die Filtration, besonders bevorzugt die Ultrafiltration, sind erfindungsgemäß zu Separation von Hefebiomasse und Fermentationslösung vorgesehen.

Nach der Separierung von der Fermentationslösung liegt die Hefebiomasse als sogenannte "Hefemilch" vor, die, bevor weiter aufbereitet, optional zur Reinigungszwecken mehrmals verdünnt und erneut separiert werden kann. Je nach gewünschter Produktform wird die Hefemilch zu Preßhefe, aktiver Trockenhefe oder Trockenhefe aufbereitet. Bei der Verarbeitung zu Preßhefe wird die Hefemilch über Filterpressen oder Vakuumrotationsfilter auf einen Trockenstoffanteil von 27-30% aufkonzentriert und anschließend die Masse durch eine Strangpresse ausgeformt und abgepackt. Bei der Verarbeitung zu aktiver Trockenhefe wird die Preßhefe im Extruder zu kleinen Zylindern geformt, die schonend thermisch, beispielsweise im Wirbelschichtverfahren getrocknet werden. Teilweise wird auch gefriergetrocknet. Bei der Weiterverarbeitung zu Trockenhefe wird die Hefemilch üblicherweise in einem Konktakttrockner, beispielsweise im Walzentrockner, oder in einem Konvektionstrockner, beispielsweise in einer Sprühtrockenanlage, getrocknet.

Erfindungswesentlich ist, daß die bei der Hefebiomassengewinnung anfallende Fermentationslösung zur Erzeugung von Biogas weiterverwendet wird und nicht, wie aus dem Stand der Technik bekannt, dem Abwassersystem zugeführt wird und damit aufgrund der organischen Belastung eine Gefahr für die Umwelt darstellt.

Bei der Biogaserzeugung werden die hochmolekularen organischen Substanzen (Eiweiß, Kohlenhydrate, Fett, Zellulose) im Wege des anaeroben Abbaus, der sogenannten Methangärung, zu niedermolekularen Verbindungen (Einfachzucker, Aminosäuren, Fettsäuren, Wasser) abgebaut, wobei durch die daran beteiligten Bakterien zum einen ein hochwertiger biologischer Dünger (Reststoffe) als auch Biogas entsteht.

Das Biogas enthält überwiegend durch Methanbakterien gebildetes Methan (ca. 60-65%) und Kohlendioxid (ca. 30%) und nur wenige Prozente anderer Gase wie Schwefelwasserstoff, Wasserstoff, Stickstoff und Ammoniak. Das Temperaturoptimum der meisten bekannten Methanbakterien liegt im mesophilen Bereich (30-40°C). Dieser Temperaturbereich ist bei dem Betrieb einer Biogasanlage auch besonders wirtschaftlich, da die Abbaudauer nicht übermäßig lang ist und nicht durch einen hohen energethischen Aufwand durch Beheizung erkauft werden muß. Eine typische Biogasanlage, wie sie beispielsweise in der Landwirtschaft eingesetzt wird, weist einen Vorbehälter zur Sammlung organischer Biomasse auf, von wo aus diese Biomasse, evtl. nach vorheriger Zerkleinerung in einer Homogenisiereinrichtung, in einem Gärbehälter gelangt wo die Biomasse in mesophilen Temperaturbereich gehalten wird. Die Biomasse kann von einem Rührwerk im Gärbehälter ständig vermischt werden, wodurch die Bildung einer festen Decke auf der Biomasse im Gärbehälter verhindert und die Biomasse besser entgast wird. Das Biogas tritt aus einer Öffnung oberhalb der gärenden Biomasse im Biogasfermentor aus und wird einem Gasspeicher zugeleitet dem ein Kondenswasserabscheider und Filter vorgelagert sein können. Die im Biogasfermentor anfallenden Reststoffe werden in ein Endlager überführt, wo sie bis zur landwirtschaftlichen Verwertung gelagert werden. Durch den Gärprozeß im Biogasfermentor werden die organischen Substanzen und Schadstoffe sicher abgebaut sowie pathogene Keime abgetötet.

Das Biogas läßt sich universell, z. B. zum Heizen, zum Trocknen oder zur Stromerzeugung einsetzen.

Die Verstromung des erzeugten Biogases erfolgt üblicherweise in einem Blockheizkraftwerk mit Zündstrahl- oder Gas-Otto-Motor. Die Abwärme der Motoren kann unmittelbar zur Erhaltung der Prozeßtemperatur im Biogasfermentor oder beispielsweise zur Erwärmung von Brauchwasser genutzt werden.

Wesentlicher Gedanke vorliegender Erfindung ist, daß das Biomasse-Abfallprodukt bei der Hefegewinnung, die Fermentationslösung, der weitere beliebige Biomassen, beispielsweise Corn Cobe Mix (CCM) Maissilage, Grassilage, Schlempe oder Getreide zugesetzt werden können, in Biogas umgesetzt wird, aus welchen elektrische und/oder thermische Energie gewonnen wird, die wiederum in das aerobe Hefegewinnungs- und Weiterverarbeitungsverfahren zurückgeführt wird.

Die aus dem Biogas gewonnene Energie läßt sich beispielsweise bei der Sterilisation und/oder Aufkonzentrierung des Prozeßwassers und/oder bei der aeroben Fermentierung und/oder bei der Weiterverarbeitung der produzierten Hefemasse beispielsweise der Autolyse und/oder der Aufkonzentrierung der erzeugten Hefebiomasse und/oder dessen Trocknung, beispielsweise der Sprüh- oder Trommeltrocknung, nutzen. Da der Trocknungsprozeß der energetisch aufwendigste und damit kostenintensivste Prozeß bei der Verarbeitung von Hefebiomasse darstellt, ist vorliegende Erfindung bei der Herstellung von Trockenhefe besonders attraktiv. Außerdem kann überschüssige, aus dem Biogas gewonnene, elektrische Energie verkauft und in das Stromnetz eingespeist werden.

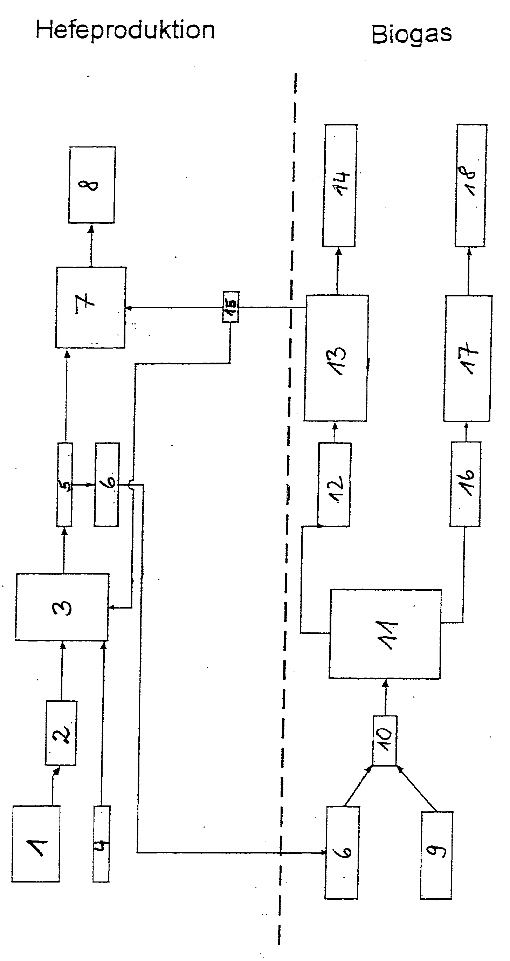

Ein Ausführungsbeispiel der Erfindung ist in der Zeichnung dargestellt und wird im Folgenden näher beschrieben.

Das Prozeßwasser sind 85.000 Tonnen Molkepermeat 1 das folgende Bestandteile aufweist:

| Bestandteile | Prozentangaben |
|---|---|
| Fett | 0,02 Gew.-% |
| Eiweiß | 0,38 Gew.-% |
| Lactose | 8,81 Gew.-% |

Der pH-Wert des Molkepermeats 1 beträgt 5,6. Das Molkepermeat 1 fällt nach der Ultrafiltration von Molke an und ist aufgrund des unsterilen Betriebs der Ultrafiltration mit Keimen belastet. Die im Molkepermeat 1 beobachteten Keimzahlen sind:
Bakterien: 3,9 x 10⁷ Kolonien bildende Einheiten/ml
Hefen: 6,9 x 10⁴ Kolonien bildende Einheiten/ml.

Um die Keimzahlen zu reduzieren wird das Molkepermeat 1 im Sterilisator 2 sterilisiert, bevor es dem Fermentor 3 zugeführt und darin mit den Hefestämmen aerob fermentiert wird. Zusätzlich werden dem Fermentor 32.000 Tonnen Supplemente 4 zugeführt, die eine optimale Fermentierung des Molkepermeats 1 durch die Hefen ermöglichen. Der Fermentierung im Fermentor 3 schließt sich die Separierung in Separator 5 an in 66.000 Tonnen Überstand 6 und Hefebiomasse, die als sogenannte Hefemilch (20.000 Tonnen) vorliegt und nach Filtrierung, Trocknung und Walzung 7 zu 4.000 Tonnen Trockenhefe 8 (93% TS) weiterverarbeitet wird. Die 66.000 Tonnen Überstand 6 der Separierung im Separator 5 werden unter Zusatz von 12.000 Tonnen Corn Cobe Mix (CCM) und 9.000 Tonnen Getreide 9 nach vorheriger Homogenisierung in einen Homogenisator 10 in einem Methanreaktor 11 abgebaut. Die dabei anfallenden 70.000 m³ Gärsubstrat 16 werden in einen Endlager 17 überführt und stehen als Pflanzendünger 18 für die Landwirtschaft zur Verfügung. Neben dem Gärsubstrat 16 wird in dem Methanreaktor 11 Biogas gebildet, das in einem 10,3 Mio. m³ Gasspeicher 12 zwischengelagert wird, bevor es in einem Blockheizkraftwerk 13 zur Gewinnung von thermischer und elektrischer Energie 14, 15 umgesetzt wird. Die gewonnene Energie 14, 15 wird in den Hefebiomassen Gewinnungs- und Weiterverarbeitungsprozeß zur Aufrechterhaltung der Fermentationstemperatur im Fermentor 3 und zur Gewinnung von Trockenhefe 8 aus der Hefemilch nach Filterung, Trocknung und Walzung 7 zumindest teilweise verwendet und überschüssige Energie 14 als elektrische Energie in das Stromnetz eingespeist.

## Patentansprüche

1. Verfahren zur Verarbeitung von Prozeßwasser, das als Hefefermentationssubstrat geeignet ist, mit den Schritten:
a) aerobe Fermentation des Prozeßwassers mit substratspezifischen Hefepopulationen,
b) Separierung des Fermentationsgemisches aus Schritt a) in Hefebiomasse und Fermentationslösung,
c) Verwendungen der in Schritt b) erhaltenen Fermentationslösung bei der Erzeugung von Biogas,
d) Verwendung des in Schritt c) erzeugten Biogases zur Gewinnung von thermischer und/oder elektrischer Energie,
e) zumindest teilweise Nutzung der in Schritt d) gewonnen Energie bei der Gewinnung und/oder Weiterverarbeitung der in Schritt b) separierten Hefebiomasse.

2. Verfahren nach Anspruch 1, **dadurch gekennzeichnet, daß** das Prozeßwasser Laktose enthält.

3. Verfahren nach Anspruch 2, **dadurch gekennzeichnet, daß** der Laktosegehalt des Prozeßwassers 0,4 Gew.%-40 Gew.%, vorzugsweise 2 Gew.%-12 Gew.%, besonders bevorzugt 3,5 Gew.%-9 Gew.% ist.

4. Verfahren nach einem der Ansprüche 1 bis 3, **dadurch gekennzeichnet, daß** das Prozeßwasser Molke und/oder Molkepermeat ist.

5. Verfahren nach einem der Ansprüche 1 bis 4, **dadurch gekennzeichnet, daß** das Prozeßwasser mit einer Phosphorquelle und/oder einer Stickstoffquelle und/oder Spurenelementen und/oder Vitaminen supplementiert ist.

6. Verfahren nach einem der Ansprüche 1 bis 5, **dadurch gekennzeichnet, daß** das Prozeßwasser sterilisiert ist.

7. Verfahren nach einem der Ansprüche 1 bis 6, **dadurch gekennzeichnet, daß** das Prozeßwasser aufkonzentriert ist.

8. Verfahren nach einem der Ansprüche 1 bis 7, **dadurch gekennzeichnet, daß** die substratspezifischen Hefepopulationen ausgewählt sind aus den Gattungen: Candida, Kluyveromyces, Debaromyces, Trichosporon, Pichia, Saccharomyces und Torolopsis.

9. Verfahren nach Anspruch 8, **dadurch gekennzeichnet, daß** die Hefen der Gattung Kluyveromyces ausgewählt sind aus den Spezien Kluyveromyces fragilis und Kluyveromyces marxianus.

10. Verfahren nach einem der Ansprüche 1 bis 9, **dadurch gekennzeichnet, daß** im Schritt a) kontinuierlich fermentiert wird.

11. Verfahren nach Anspruch 10, **dadurch gekennzeichnet, daß** bei einer Temperatur von 20-40 °c, vorzugsweise 25-35 °c, besonders bevorzugt 29-31 °c und/oder einem pH < 4 und/oder einer Durchflußrate von 1-48 Stunden, bevorzugt 5-24 Stunden, besonders bevorzugt 8-12 Stunden fermentiert wird.

12. Verfahren nach einem der Ansprüche 1 bis 9, **dadurch gekennzeichnet, daß** im Schritt a) diskontinuierlich (Batch-Fermentation) fermentiert wird.

13. Verfahren nach einem der Ansprüche 1 bis 12, **dadurch gekennzeichnet, daß** die Separierung des Fermentationsgemisches in Schritt b) durch Filtration, bevorzugt Ultrafiltration erfolgt.

14. Verfahren nach einem der Ansprüche 1 bis 13, **dadurch gekennzeichnet, daß** die separierte Fermentationslösung aus Schritt b) mit CCM und/oder Getreide zu Biogas umgesetzt wird.

15. Verfahren nach einem der Ansprüche 1 bis 14, **dadurch gekennzeichnet, daß** die Hefebiomasse autolysiert wird.

16. Verfahren nach einem der Ansprüche 1 bis 15, **dadurch gekennzeichnet, daß** die Hefebiomasse aufkonzentriert wird.

17. Verfahren nach einem der Ansprüche 1 bis 16, **dadurch gekennzeichnet, daß** die im Schritt d) gewonnene Energie bei der Sterilisation und/oder Aufkonzentrierung des Prozeßwassers und/oder bei der Fermentierung und/oder bei der Autolyse und/oder der Aufkonzentrierung der Hefebiomasse genutzt wird.

18. Verfahren nach einem der Ansprüche 1 bis 17, **dadurch gekennzeichnet, daß** die in Schritt d) gewonnene Energie bei der Trocknung der Hefebiomasse genutzt wird.
